(19) Europäisches Patentamt / European Patent Office / Office européen des brevets

(11) **EP 1 244 912 B1**

(12) **FASCICULE DE BREVET EUROPEEN**

(45) Date de publication et mention
de la délivrance du brevet:
**23.05.2007 Bulletin 2007/21**

(51) Int Cl.:
*G01N 33/50* (2006.01)  *C12Q 1/68* (2006.01)
*C12N 7/02* (2006.01)

(21) Numéro de dépôt: **00985423.3**

(22) Date de dépôt: **13.12.2000**

(86) Numéro de dépôt international:
**PCT/FR2000/003503**

(87) Numéro de publication internationale:
**WO 2001/044809 (21.06.2001 Gazette 2001/25)**

(54) **EVALUATION D'AGENTS BIOLOGIQUES DANS DES CELLULES CIBLES VIVANTES**

AUSWERTUNG VON BIOLOGISCHEN MITTELN IN LEBENDEN ZIELZELLEN

METHODS FOR SCREENING OR EVALUATING THE PERFORMANCE OF A SET OF BIOLOGICAL
AGENTS IN LIVING TARGET CELLS AND THEIR USES

(84) Etats contractants désignés:
**AT BE CH CY DE DK ES FI FR GB GR IE IT LI LU
MC NL PT SE TR**

(30) Priorité: **16.12.1999 FR 9915884**

(43) Date de publication de la demande:
**02.10.2002 Bulletin 2002/40**

(73) Titulaire: **Nautilus Biotech
91000 Evry Cédex (FR)**

(72) Inventeur: **VEGA, Manuel
F-91270 Vigneux-sur-Seine (FR)**

(74) Mandataire: **Goulard, Sophie et al
Cabinet ORES
36, rue de St Pétersbourg
75008 Paris Cedex (FR)**

(56) Documents cités:
• CHARBORD P; NEEL H; LEHN P; PARMENTIER C: "NORMAL HUMAN GRANULO MONOCYTIC BONE MARROW PROGENITOR CELLS RESPONSIVENESS TO COLONY STIMULATING ACTIVITY" NOUVELLE REVUE FRANCAISE D'HEMATOLOGIE, vol. 22, 1980, pages 357-370, XP000949859

• SCHUHMANN KLAUS; ROMANIN CHRISTOPH; BAUMGARTNER WERNER; GROSCHNER KLAUS: "Intracellular Ca2+ inhibits smooth muscle L-type Ca2+ channels by activation of protein phosphatase type 2B and by direct interaction with the channel" JOURNAL OF GENERAL PHYSIOLOGY, vol. 110, novembre 1997 (1997-11), pages 503-513, XP000949861

• MOULLIER P; DAVELOOSE D; LETERRIER F; HOEBEKE J: "COMPARATIVE BINDING OF WHEAT GERM AGGLUTININ AND ITS SUCCINYLATED FORM ON LYMPHOCYTES" EUROPEAN JOURNAL OF BIOCHEMISTRY, vol. 161, 1986, pages 197-204, XP000951462

• ATKINSON E M ET AL: "A high-throughput hybridization method for titer determination of viruses and gene therapy vectors" NUCLEIC ACIDS RESEARCH,GB,OXFORD UNIVERSITY PRESS, SURREY, vol. 26, no. 11, 1 juin 1998 (1998-06-01), pages 2821-2823, XP002099502 ISSN: 0305-1048 cité dans la demande

• NELSON DAVID M; WAHLFORS J JARMO; CHEN LIN; ONODERA MASAFUMI; MORGAN RICHARD A: "Characterization of diverse viral vector preparations, using a simple and rapid whole-virion dot-blot method" HUMAN GENE THERAPY, vol. 9, 1 novembre 1998 (1998-11-01), pages 2401-2405, XP000951429 cité dans la demande

• MITTEREDER NANETTE; MARCH KEITH L; TRAPNELL BRUCE C: "Evaluation of the concentration and bioactivity of adenovirus vectors for gene therapy" JOURNAL OF VIROLOGY, vol. 70, no. 11, novembre 1996 (1996-11), pages 7498-7509, XP002148995 cité dans la demande

**(Cont. page suivante)**

EP 1 244 912 B1

- **SALVETTI A ET AL: "Factors influencing recombinant adeno-associated virus production" HUMAN GENE THERAPY, XX, XX, vol. 9, no. 5, 20 mars 1998 (1998-03-20), pages 695-706, XP002097712 ISSN: 1043-0342 cité dans la demande**

- **DAVIS A R ET AL: "HIGH THROUGHPUT METHOD FOR CREATING AND SCREENING RECOMBINANT ADENOVIRUSES" GENE THERAPY, GB, MACMILLAN PRESS LTD., BASINGSTOKE, vol. 5, no. 8, août 1998 (1998-08), pages 1148-1152, XP000867556 ISSN: 0969-7128 cité dans la demande**

**Description**

L'invention est relative à des méthodes de criblage et/ou d'évaluation de la performance d'un ensemble d'agents biologiques, tels qu'une banque de vecteurs viraux ou non viraux recombinants (vecteurs de transfert de gènes), de vaccins, de protéines recombinantes ou d'anticorps, dans des cellules cibles vivantes (système biologique complexe).

Les progrès relatifs aux transferts de gènes en thérapie génique dépendent de la capacité à développer des vecteurs permettant l'expression d'une substance au niveau de la cellule cible, ladite substance ayant effectivement un effet thérapeutique au niveau de ladite cible. Il est donc important de pouvoir disposer de vecteurs de qualité clinique, utilisables dans des essais de phase I.

Pour l'obtention d'une information quantitative concernant les performances potentielles d'un vecteur de transfert de gène, les paramètres d'évaluation proposés dans la littérature sont :

. le titre en particules physiques (*pp*) (Mittereder et al., J. Virol., 1996, 70, 11, 7498-7509 ; Atkinson et al., NAR, 1998, 26, 11, 2821-2823 ; Kechli et al., Hum. Gene Ther., 1998, 9, 4, 587-590 ; Nelson et al., Hum. Gene Ther., 1998, 9, 16, 2401-2405), qui représente le contenu total en particules de vecteur; habituellement ce titre est évalué soit à partir du contenu en acides nucléiques des vecteurs (hybridation des acides nucléiques ou $DO_{260}$ respectivement pour AAV et AdV), soit à partir du contenu en protéines virales (activité RT et contenu en p24, par exemple pour MLV et HIV, respectivement) ; la mesure physique des particules virales ou des génomes présente l'inconvénient de pouvoir être confondue avec la présence de particules défectives *(defective-interfering particles ou DI)* et

. le titre en particules infectieuses *(ip* : unités infectieuses, unités de transduction) (Mittereder et al., précité ; Weitzman. et al., J. Virol., 1996, 70, 3, 1845-1854 ; Salvetti et al., Hum. Gene Ther., 1998, 9, 5, 695-706) est évalué par l'étude des changements observés dans les cellules infectées (réplication virale, intégration du provirus, lyse cellulaire, expression du transgène), par des méthodes essentiellement basées sur les dilutions en série, suivies soit par une extrapolation linéaire, soit par une approximation asymptotique ; ainsi *ip* mesure le nombre de particules actives dans le processus dont l'effet est mesuré : *ip* ne correspond donc pas à toutes les particules potentiellement actives ; *ip* constitue une partie des particules physiques *(pp),* l'autre partie desdites particules physiques étant constituée par des particules inactives *(nip* ou *non-infectious particles),*

. la détection des plaques, dans le cas de virus lytiques ; cette méthode est quantitative mais difficile à mettre en oeuvre et ne peut pas s'appliquer aux virus non-cytopathiques et

. la variation des rapports particules/infectivité.

Par exemple, dans l'article au nom d'E.M. Atkinson et al. (NAR, 1998, 26, 11, 2821-2823) et dans la Demande Internationale PCT WO 99/11764, pour résoudre le problème de la détermination du titre et la comparaison de différents virus recombinants utilisés en thérapie génique, il est proposé une nouvelle méthode, considérée comme plus fiable que celles antérieurement utilisées et qui met en oeuvre essentiellement une étape d'amplification du matériel génétique viral dans une lignée cellulaire hôte, des préparations de vecteur standard de titre connu obtenues par dilutions en série et un contrôle interne de titre connu. De manière plus précise, la méthode comprend dans différents puits d'une plaque de microtitration, l'infection de cellules à l'aide d'une préparation virale, la réplication du génome viral dans ladite cellule hôte, la lyse chimique de ladite cellule, une hybridation de l'acide nucléique puis la détermination de la quantité relative d'acide nucléique viral répliqué dans chaque puits.

Dans l'article au nom d'A.R. DAVIS et al. (Gene Therapy, 1998, 5, 1148-1152), il est considéré qu'une utilisation optimale des adénovirus recombinants en thérapie génique passe par le développement d'une technique adaptée à la séparation rapide de nouveaux recombinants qui ne sont pas contaminés par le virus sauvage. Pour ce faire, les Auteurs de cet article proposent de créer des adénovirus recombinants en cotransfectant des cellules 293 avec l'ADN viral dérivé à partir de la région 3' du génome d'un recombinant qui ne comprend pas la région El mais qui exprime la protéine fluorescente (GFP ou *green fluorescent protein*) et un plasmide portant la région 5' du génome; la cotransfection peut ainsi être visualisée par microscopie de fluorescence.

Les méthodes de l'art antérieur font appel exclusivement à la mesure du titre en particules physiques *(pp)* et/ou à la mesure du titre en particules infectieuses (*ip*) pour évaluer un vecteur de transfert de gène. Les préparations de vecteur avec un titre élevé en particules infectieuses et un rapport particules physiques/particules infectieuses faible sont considérées comme étant de haute qualité, ces deux paramètres étant considérés comme fournissant une information quantitative concernant la performance d'un vecteur de transfert de gène.

Toutefois, les procédures actuelles utilisées pour évaluer *pp* ainsi que *ip* varient selon le type de vecteur, sont peu reproductibles et imprécises ; en outre, ces paramètres ne sont pas suffisamment informatifs pour permettre de définir de manière précise les caractéristiques d'un vecteur et donc d'évaluer ses performances.

La présente invention s'est en conséquence donnée pour but, de fournir un procédé standardisé qui soit apte à évaluer l'interaction entre un vecteur de thérapie génique et plus généralement de n'importe quel agent biologique, avec un système biologique complexe (cellules cibles vivantes).

C'est également un but de l'invention de fournir un procédé de criblage d'un ensemble d'agents biologiques complexes, en vue de la sélection de l'agent biologique le plus adapté à l'application recherchée.

La présente invention a pour objet un procédé d'évaluation de la performance d'un ensemble d'agents biologiques complexes dans des cellules cibles vivantes, avec lesquelles lesdits agents biologiques interagissent, lequel procédé est caractérisé en ce qu'il comprend au moins les étapes suivantes :

(a) la préparation, pour chaque agent biologique dudit ensemble, d'une gamme d'échantillons, obtenue par dilutions en série dudit agent biologique à une concentration R1,

(b) l'incubation de chaque échantillon de ladite gamme de dilutions obtenue en 1. avec lesdites cellules cibles à une concentration constante R2,

(c) la détermination du produit P de la réaction R1 + R2, à un instant t, dans chacun desdits échantillons,

(d) l'établissement d'une courbe théorique H à partir desdits points expérimentaux R1 et P, pour chaque agent biologique par approximation itérative de paramètres qui reflètent la réaction R1+R2 → P, audit instant t, conformément à l'équation suivante :

$$P = P_{max} \, (\pi R1)^{r} / (\kappa + (\pi R1)^{r}) \qquad r=1,\ldots,n \qquad (2),$$

dans laquelle :

R1 représente la concentration en agent biologique dans un échantillon de la gamme,
P représente le produit de la réaction R1 + R2 à un instant t,
$P_{max}$ représente la capacité maximale de la réaction,
$\kappa$ représente la résistance du système biologique à une concentration constante R2 à répondre audit agent biologique (constance de résistance de R2),
r représente un coefficient qui dépend de R1 et qui correspond au coefficient de Hill, et
$\pi$ représente la puissance intrinsèque de l'agent biologique R1 à induire une réponse dans le système biologique (production de P à l'instant t), et

(e) le tri des valeurs de $\kappa$ et de $\pi$ obtenues en (d), pour chaque agent biologique et le classement des agents biologiques en fonction desdites valeurs.

La présente invention a également pour objet un procédé de criblage d'un ensemble d'agents biologiques complexes modifiés (banque de mutants) dans des cellules cibles vivantes avec lesquelles lesdits agents biologiques interagissent, lequel procédé est caractérisé en ce qu'il comprend au moins les étapes (a) à (e) telles que définies ci-dessus et une étape (f) de sélection de l'agent biologique le plus adapté à l'application recherchée.

Selon un mode de mise en oeuvre avantageux dudit procédé, l'ensemble d'agents biologiques modifiés est constitué par une banque de mutants, obtenue par l'introduction naturelle ou artificielle d'une ou plusieurs mutations dans la séquence nucléotidique et/ou peptidique desdits agents biologiques.

Au sens de la présente invention, on entend par mutation, une insertion, une délétion ou une substitution d'au moins un nucléotide ou d'au moins acide aminé.

Les procédés selon l'invention consistent à analyser pour chaque gamme d'agent biologique à tester, sur la base de l'équation de Hill, la réponse du système biologique (production d'un produit P à un instant t).

L'équation de Hill est une formalisation générale qui décrit l'interaction entre différentes molécules. Elle exprime la quantité de produit formé comme une fonction de la concentration des réactifs et de la constante d'affinité du système.

Originellement développée pour l'étude de la dissociation entre l'hémoglobine et l'oxygène, l'équation de Hill englobe l'analyse de la cinétique enzymatique par l'équation de Michaelis-Menten, l'analyse de la liaison ligand-récepteur et l'analyse des systèmes allostériques.

En effet, conformément à Hill, pour une réaction simple :

$$R1 + R2 \xrightarrow{K} P$$

dans laquelle l'affinité K entre R1 et R2 change en fonction de leurs concentrations ; l'équation de Hill décrit l'accumulation du produit P comme une fonction de la concentration de l'un des réactifs (R1) et des propriétés intrinsèques (K) du système.

$$P = \sum_{r=1}^{r=n} P_{max} \cdot R1^r / (K + R1)^r \qquad (R2 \text{ constante}) \quad (1)$$

dans laquelle R1, P, $P_{max}$ et K représentent respectivement la concentration du réactif R1, la concentration du produit P, la capacité maximale de la réaction et la constante d'affinité entre R1 et R2.

Le coefficient de Hill r est une fonction de R1. r est égal à 1 lorsque des sites de liaison interactifs indépendants sont impliquées entre R1 et R2, comme dans le cas décrit par Michaelis-Menten. r varie de 1 à n pour des systèmes dans les lesquels les sites impliqués dans l'interaction entre R1 et R2 ne sont pas indépendants les uns des autres. L'affinité pour R1 au niveau de n'importe quel site de liaison de R2 varie comme une fonction soit du degré d'occupation des autres sites de R2, soit de la concentration de R1 lui-même ou soit de la concentration d'autres régulateurs (positifs ou négatifs).

Ces analyses basées sur l'équation de Hill se sont toujours limitées à des protéines individuelles ou à des systèmes simples mis en oeuvre dans des conditions expérimentales définies de manière très précise.

Il n'était pas pensable, jusqu'à présent que l'équation de Hill pouvait s'appliquer à l'interaction entre des systèmes complexes telles que les cellules vivantes et des agents biologiques complexes tels que les virus, les vecteurs viraux et non-viraux recombinants comme les vecteurs de transfert de gènes, les vaccins, les protéines recombinantes ou les anticorps, par exemple.

De manière surprenante, l'Inventeur a maintenant trouvé que l'analyse de systèmes complexes tels que l'infection d'une cellule par un virus ou un vecteur de thérapie génique, était possible par sélection de paramètres directement dérivés de l'équation de Hill.

**Premières définitions**

- R1 correspond à la concentration en agent biologique; il peut signifier, dans la présente invention, selon le contexte, l'ensemble des concentrations obtenues par dilution de la préparation d'agent biologique, utilisées pour déterminer le produit P ou l'agent biologique lui-même ;
- on entend par agent biologique, par exemple, mais de manière non limitative, un vecteur viral ou non viral recombinant contenant une molécule d'acide nucléique d'intérêt (gène, cassette d'expression d'une protéine, molécule d'ARN anti-sens, ribozyme, génome viral recombinant ou fragment de ce génome) tel qu'un vecteur de transfert de gènes ou un vecteur d'expression, un virus, un anticorps, un vaccin ou une protéine recombinante ;
- R2 correspond à la concentration des cellules cibles vivantes, il peut signifier, dans la présente invention, selon le contexte, la concentration des cellules cibles vivante (constante) utilisée pour déterminer le produit P ou les cellules cibles vivantes elles-mêmes ;
- on entend par cellules cibles vivantes, des cellules cibles *in vivo, in vitro* ou *ex vivo,* avant leur modification par un agent biologique ;
- P *(output)* représente la réponse des cellules cibles R2 à chaque dilution d'agent biologique *(input)* à une concentration R1; le produit P peut être déterminé soit directement, soit indirectement par la mesure de paramètres biologiques reflétant la réponse du système biologique (cellules cibles) audit agent biologique (ou réaction R1+R2 ou processus biologique) ; il s'agit notamment de la mesure d'une activité enzymatique, de l'expression d'un transgène, de la productivité d'un virus, de la cytotoxicité, de la tumorigenèse, de l'immunogénicité etc..... Dans ce cas, le test biologique mis en oeuvre pour déterminer P est soit un test *in vitro,* soit un test *in vivo;* il permet de déterminer des paramètres biologiques représentatifs de la réponse du système biologique à l'agent biologique étudié ;
- les techniques utilisées pour déterminer, estimer, analyser ou calculer les valeurs de P à un instant t sont, de manière non limitative, des mesures de radioactivité, de fluorescence, de luminenescence, d'absorbance ou le dénombrement de cellules ;
- le paramètre $\pi$ : $\pi$ mesure la puissance intrinsèque de l'agent biologique pour produire P dans les cellules cibles vivantes considérées ; $\pi$ s'oppose à $\kappa$ (constante de résistance) qui constitue le facteur d'opposition desdites cellules à la production de P ; par exemple, dans le cas où l'agent biologique est représenté par des particules virales infectieuses (R1), l'on peut considérer que pour chaque particule virale infectieuse ajoutée, l'activité du virus est donnée par l'équation $\pi$R1 ; pour obtenir une réponse des cellules cibles vivantes (production de P), la puissance intrinsèque $\pi$ doit être supérieure à $\kappa$ dans la cellule. $\pi$ constitue une caractéristique spécifique de l'agent biologique étudié ; dans ce contexte, des variants d'un agent biologique étudié ne présenteront pas la même valeur de $\pi$ dans un processus réactionnel donné. On peut considérer que $\pi$ constitue un paramètre reflétant l'activité chimique par opposition à la concentration pour des composés chimiques simples. $\pi$ constitue un facteur de correction qui affecte la concentration R1 de l'agent biologique pour indiquer sa force ou activité réelle dans un processus réactionnel donné ; les variations de $\pi$ affectent l'équation (2) en déplaçant la courbe vers la droite ou vers la gauche selon que la valeur de $\pi$ décroît ou croît ; la pente de la courbe obtenue à l'étape (d) augmente, lorsque $\pi$ augmente ; les courbes obtenues à l'étape (d), qui ne diffèrent entre elles qu'en ce qui concerne le paramètre $\pi$, ne sont pas parallèles entre elles; $\pi$ est un paramètre clé pour la caractérisation de l'agent biologique et la détermination de sa performance pour accomplir la réaction (processus biologique) étudiée: $\pi$ trouve une application directe et pratique dans l'optimisation et la mise au point de l'agent biologique utilisé, dans la mesure où ce paramètre permet de comparer la puissance relative de variants dudit agent ; toutefois, ce paramètre ne permet pas seul d'évaluer l'ensemble du système ;

- le paramètre $\kappa$ (constante de résistance) : $\kappa$ mesure la résistance interne des cellules cibles vivantes au processus biologique induit par l'agent biologique pour l'obtention de P ; $\kappa$ est une caractéristique spécifique du processus biologique (réaction entre R1 et R2) particulier et des cellules cibles vivantes (type cellulaire) testées : le même processus biologique pour des lignées ou des types cellulaires différents conduira à l'obtention de paramètres $\kappa$ différents ; de plus, les facteurs qui ont une influence sur la performance d'une cellule dans la réalisation dudit processus biologique, tels que les agents contaminants ou les agents toxiques, modifient la valeur de $\kappa$ pour ladite cellule ; on peut considérer que $\kappa$ est analogue aux constantes de dissociation ou d'affinité pour des composés chimiques ou des réactions biologiques simples ; les variations de $\kappa$ affectent l'équation (2) en déplaçant la courbe de la droite vers la gauche, selon que $\kappa$ augmente ou diminue ; les courbes obtenues à l'étape (d), qui ne diffèrent entre elles qu'en ce qui concerne le paramètre $\kappa$, sont parallèles entre elles ; $\kappa$ est un paramètre clé pour l'estimation des performances du test biologique sélectionné pour évaluer la réaction globale (réaction R1+R2) ; $\kappa$ trouve une application directe et pratique dans le développement et la validation du test sélectionné pour évaluer la réaction dans laquelle intervient l'agent biologique et dans l'évaluation de la susceptibilité ou la sensibilité de différents types cellulaires à participer à ladite réaction.

De manière surprenante, conformément au procédé selon l'invention, différents agents biologiques complexes et/ou différentes cellules cibles vivantes peuvent être comparés et classés sur la base de leur performance évaluée par au moins les deux paramètres ci-dessus mentionnés, désignés par l'expression « paramètres de Hill ».

Ainsi une analyse précise et une comparaison de la réponse biologique des cellules cibles vivantes tant *in vitro* qu'*in vivo* à des agents biologiques complexes devient effectivement possible.

Également conformément à l'invention un certain nombre de paramètres, dérivés de l'équation de Hill, peut être enregistré et utilisé pour quantifier des caractéristiques pertinentes d'un système complexe : agent biologique, cellules cibles et processus ou réaction, résultant de leur interaction.

Par exemple, dans le cas où le système complexe serait représenté par l'infection d'une cellule par un virus, on peut observer un grand nombre d'interactions (protéine/protéine, protéine/acide nucléique, protéine/petite molécule), qui sont susceptibles d'être, conformément à l'invention, décrites par le procédé mettant en oeuvre l'équation de Hill. La réaction globale, formalisée par les réactifs entrants *(input)*, à savoir les virus et les cellules et les produits de la réaction *(output)* [réponse cellulaire à l'infection] peut être analysée en utilisant l'équation de Hill, et ce, quel que soit le nombre d'étapes intermédiaires.

Selon un mode de mise en oeuvre avantageux desdits procédés, l'agent biologique est sélectionné dans le groupe constitué par les virus, les vecteurs viraux et non-viraux, les vaccins, les anticorps et les protéines recombinantes.

Des réactions complexes, telles que celles impliquant l'interaction entre des virus recombinants (R1) et des cellules vivantes (R2) et qui induisent une réponse biologique, peuvent être analysées et les réactifs (R1 et R2) caractérisés par le procédé selon l'invention mettant en oeuvre l'équation de Hill (paramètres $\pi$ et $\kappa$) et éventuellement au moins l'un des paramètres dérivés, tels que définis ci-après.

Selon un autre mode de mise en oeuvre avantageux desdits procédés, à l'étape (c), la détermination de P est réalisée soit de manière directe, par exemple par dosage de P, soit de manière indirecte, par exemple, à l'aide d'un test biologique convenablement sélectionné pour la mesure d'au moins un paramètre ou une variable reflétant la réponse des cellules cibles vivantes audit agent biologique, comme précisé ci-dessus.

Selon un autre mode de mise en oeuvre avantageux desdits procédés, ils comprennent, en outre, la mesure d'au moins l'un des paramètres dérivés suivants :

- l'efficacité globale $\varepsilon$ de la réaction induite par l'agent biologique sur ledit système,
- le titre apparent $\tau$ de l'agent biologique,
- le titre absolu $\theta$ dudit agent biologique, et
- l'index d'hétérogénéité $\eta$ de ladite réaction biologique.

***Définitions de ces paramètres dérivés***

- efficacité globale $\varepsilon$ : $\varepsilon$ mesure l'efficacité globale maximale de la réaction agent biologique (R1), caractérisé par un paramètre $\pi$ donné et des cellules cibles vivantes (R2), caractérisées par un paramètre $\kappa$ donné : $\varepsilon$ est donc spécifique du couple agent biologique ($\pi$)/système biologique ($\kappa$) pour ce qui concerne la réaction étudiée ; des modifications des paramètres $\pi$ et/ou $\kappa$ entraînent des modifications du paramètre $\varepsilon$. $\varepsilon$ est un paramètre clé pour la caractérisation de l'efficacité de la réaction globale mettant en oeuvre R1 et R2 ; il est en particulier important et utile pour l'optimisation du ***test*** lorsque $\pi$ et $\kappa$ ont été sélectionnés et pour étudier de manière séparée les modifications soit de $\pi$, soit de $\kappa$ ;

- le titre apparent $\tau$ : dans l'équation de Hill (2), lorsque R1 augmente, r augmente de 1 à 2, 3, 4 .... et P approche sa valeur maximale $P_{max}$. Dans l'autre direction, R1 ne peut diminuer que jusqu'à un point minimal ($R1_{min}$), auquel correspondent les valeurs minimales de r et de P. La courbe sigmoïdale de Hill n'est pas symétrique : seul son bras droit est asymptotique (jusqu'à $P_{max}$) ; sur son bras gauche, la courbe a une origine à $R1_{min}$. D'un point de vue biologique, le fait qu'il n'existe pas de P pour R1 < $R1_{min}$, signifie qu'il n'y a pas de produit de réaction lorsque la concentration en agent biologique est < $R1_{min}$: le système ne répond pas à des valeurs de R1 < $R1_{min}$. $R1_{min}$ représente donc la quantité minimale de R1 qui induit une réponse dans les cellules cibles vivantes concernées et est représenté par $\tau$ ; le titre, défini de cette manière, ne correspond ni à une valeur asymptotique, ni à une valeur approchée par extrapolation, mais un paramètre précis de l'équation de Hill et correspond à l'origine mathématique de la courbe théorique obtenue en (d). On peut donc considérer que $\tau$ mesure la dilution limite ou le titre apparent de l'agent biologique étudié ; la valeur de $\tau$ est déterminée par la limite de sensibilité du système et par la méthode utilisée pour mesurer le produit P ; c'est la raison pour laquelle il est dénommé titre apparent ; $\tau$ est spécifique de la quantité d'agent biologique testé et représente la concentration apparente de l'agent biologique ; il est exprimé en unités par volume (dilution maximale d'agent biologique qui induit la production de P). En d'autres termes, $\tau$ est représenté par le R1 maximal pour lequel le coefficient de Hill r atteint sa valeur minimale, ledit coefficient de Hill devenant constant pour une valeur égale à ou proche de 1. $\tau$ selon la présente invention, correspond au titre généralement utilisé pour les virus, les anticorps et les vecteurs ; toutefois, contrairement à ce qui est décrit dans l'art antérieur, ce paramètre seul ne permet pas d'évaluer un système biologique complexe. Les variations de $\tau$ affectent l'équation (2) en déplaçant la courbe vers la droite ou vers la gauche, selon que la valeur de $\tau$ décroît ou croît, respectivement. $\tau$ constitue un paramètre clé qui mesure la concentration apparente du stock initial d'agent biologique, qui est nécessaire à l'usage que l'on souhaite en faire;

- titre absolu $\theta$ : $\theta$ mesure le titre absolu en agent biologique ; la valeur de $\theta$ n'est ni déterminée, ni dépendante de la limite de sensibilité des cellules cibles testées ou de la méthode utilisée pour mesurer P ; c'est la raison pour laquelle $\theta$ est dénommé titre absolu ; $\theta$ est spécifique de la quantité initiale d'agent biologique testé ; il représente la concentration physique réelle en agent biologique et est exprimé en unités/volume (i. e. la dilution maximale d'agent biologique qui induit la production de P) ; $\theta$ est obtenu, conformément à l'équation (3) suivante :

$$\theta \, \pi = \tau /s \qquad\qquad\qquad (3),$$

dans laquelle s représente la sensibilité de la méthode de détection.
En conséquence, pour des agents biologiques évalués en utilisant la même méthode de détection de P, l'expression suivante, représentée dans l'équation (4) suivante est valable :

$$\theta 1 \pi 1 \, / \, \tau 1 = \theta 2 \pi 2 \, / \, \tau 2 = \theta n \pi n \, / \, \tau n = \text{constante} \qquad\qquad (4).$$

En utilisant l'équation (4), le rapport entre deux titres absolus correspondant à deux préparations d'agents biologiques différents, peut être obtenu à partir des valeurs de $\pi$ et de $\tau$ de ces deux préparations d'agents biologiques. Les variations de $\theta$ affectent l'équation (2) en déplaçant la courbe vers la droite ou vers la gauche et/ou en changeant la pente de la courbe. $\theta$ est le paramètre qui mesure la concentration absolue initiale en agent biologique.

- l'index d'hétérogénéité $\eta$ : $\eta$ mesure l'hétérogénéité interne de la réaction qui peut être due, soit aux cellules (discontinuité de la constante de résistance $\kappa$), soit à l'agent biologique (discontinuité de la puissance intrinsèque $\pi$). La présence d'une hétérogénéité interne dans la réaction R1+R2 peut être détectée par l'apparition de paliers dans l'évolution du coefficient de Hill, correspondant à la courbe de Hill qui s'ajuste aux données expérimentales. η est donc défini comme l'index d'hétérogénéité et sa valeur correspond au nombre de paliers observés dans l'évolution du coefficient de Hill : un palier : η = 1 ; deux paliers : η = 2 ; trois paliers : η = 3 ; n paliers : η = n. η est un paramètre clé pour l'analyse détaillée de la réaction. Il est utile pour l'étude de chaque palier identifié. La présence de paliers se traduit par une discontinuité brusque de κ ou de π. Les processus complexes, tels que ceux évalués dans la présente invention, consistent en une succession d'événements dans un réseau multidimensionnel de réactions biologiques reliées entre elles et interrégulées. Ainsi, la constante de résistance κ pour une réaction particulière est un indicateur macroscopique de la résistance globale de la réaction biologique ($\kappa = \kappa_1 \times \kappa_2 \times \kappa_i ... \kappa_n$). Si la contribution des constantes microscopiques de résistance ($\kappa_1$, $\kappa_2$, $...\kappa_i,...\kappa_n$) pour les paliers individuels impliqués dans la réaction étaient homogènes, et qu'aucun seuil n'existait pour passer d'un palier à l'autre ; dans ce cas, on n'observerait aucune discontinuité dans l'évolution du coefficient de Hill par rapport à R1. Cependant, l'existence d'une hétérogénéité importante parmi les valeurs $\kappa_1$ correspondant aux paliers individuels microscopiques pourrait conduire à une discontinuité macroscopique du système. Cette hétérogénéité pourrait entraîner des modifications dans la variation du coefficient de Hill et en conséquence, le besoin d'un saut quantitatif des valeurs macroscopiques de κ afin que l'équation (2) s'ajuste aux données..

En conséquence, chaque palier peut-être déterminé par une constante de résistance macroscopique κ différente et/ou une constante de résistance macroscopique π différente. Les systèmes dans lesquels η = 2 peuvent ainsi être décrits à l'aide d'une équation de Hill dans laquelle :

- κ prend deux valeurs différentes : κ1 et κ2, selon les valeurs de R1 considérées : une partie de la courbe est décrite par κ1 et une autre partie de la courbe est décrite par κ2. Les courbes de Hill décrivant la réaction globale, caractérisées par η = 2 sont des hybrides générées à partir de deux courbes de Hill parallèles différant uniquement par le paramètre κ. La transition d'une courbe à l'autre peut modifier la pente de la courbe de Hill résultante ou
- π prend deux valeurs différentes : π1 et π2, selon les valeurs de R1 considérées : une partie de la courbe est décrite par π1 et une autre partie de la courbe est décrite par π2. Les courbes de Hill décrivant la réaction globale, caractérisées par η = 2 sont des hybrides générées à partir de deux courbes de Hill parallèles différant uniquement par le paramètre π. La transition d'une courbe à l'autre peut modifier la pente de la courbe de Hill résultante.

Selon un autre mode de mise en oeuvre avantageux desdits procédés, la mesure du paramètre d'évaluation ε représentant l'efficacité spécifique d'un agent biologique apte à induire la production de P dans lesdites cellules cibles vivantes est réalisée:

- soit par mesure de la pente, de la courbe H théorique obtenue en (d), à son point d'inflexion,
- soit par calcul du maximum de la dérivée première $\delta P/\delta R1$ et éventuellement de la dérivée seconde de la courbe théorique H obtenue en (d) ; en effet, l'efficacité de la réaction décrite dans l'équation (2) est donnée par l'augmentation de *l'output* P qui peut être obtenue en augmentant *l'input* R1. Ainsi, la dérivée première de P par rapport à R1, ou la pente de la courbe décrite dans l'équation (2) fournit l'efficacité globale de la réaction pour chaque *input* ou entrée R1.

L'efficacité globale maximale ou ε est donc bien exprimée directement soit par la pente au point d'inflexion de la courbe décrite dans l'équation (2), soit par le maximum de sa dérivée $\delta P/\delta R1$. A la fois la pente de la courbe donnée par l'équation (2) et le maximum de $\delta P/\delta R1$ augmentent lorsque ε augmente.

Selon un autre mode de mise en oeuvre avantageux desdits procédés, ils comprennent, en outre, la mesure des paramètres suivants : $\pi/P_{max}$, $\kappa/P_{max}$ ou $\epsilon/P_{max}$ Ces valeurs corrigées sont indépendantes de la capacité maximale ($P_{max}$), elles permettent donc de mieux comparer les différents paramètres, π, κ et ε dans le cas où $P_{max}$ serait différente selon les systèmes ou bien dans le cas où l'agent biologique affecterait $P_{max}$.

Selon encore un autre mode de mise en oeuvre desdits procédés, les valeurs des paramètres de Hill correspondant à chaque agent biologique sont comparées à celles obtenues avec un agent biologique de référence.

Également conformément à l'invention, pour valider l'analyse de la réaction R1+R2 par l'équation de Hill, lesdits procédés peuvent comprendre en outre une étape de traitement des données expérimentales obtenues à l'étape (d) *(Hill plot),* conformément à l'équation suivante :

$$\log | P/(1 - P) | \ \text{vs.} \ \log R1.$$

Également conformément à l'invention, la sélection des agents biologiques, des vecteurs par exemple, présentant des valeurs minimales acceptables pour les paramètres sélectionnés : $\kappa$, r, $\varepsilon$, $\theta$, $\tau$, $\eta$, $\pi/P_{max}$, $\kappa/P_{max}$ ou $\varepsilon/P_{max}$ peuvent avantageusement être soumis à une analyse itérative pour obtenir la courbe H la mieux ajustée statistiquement aux valeurs expérimentales R1 et P.

Les agents biologiques, sélectionnés, conformément aux procédés selon l'invention sont ensuite validés définitivement pour leurs propriétés biologiques.

De manière avantageuse, les paramètres $\kappa$, r, $\varepsilon$, $\theta$, $\tau$, $\eta$, $\pi/P_{max}$, $\kappa/P_{max}$ ou $\varepsilon/P_{max}$, obtenus conformément aux procédés selon l'invention sont utilisés pour:

- valider et optimiser les agents biologiques utilisables dans une application particulière (procédé de criblage d'une banque d'agents biologiques modifiés),
- développer et optimiser les tests de caractérisation d'agents biologiques, éventuellement modifiés (procédé d'évaluation de la performance).

A titre d'exemple et de façon non limitative, les procédés selon l'invention peuvent être utilisés pour :

- • cribler une banque de vecteurs viraux ou non viraux pour la thérapie génique, une banque d'anticorps pour le diagnostic d'une infection ou la sélection d'anticorps efficaces contre des cellules tumorales ou bien une banque de protéines recombinantes pour le diagnostic ou le traitement d'une pathologie humaine ou animale ;
- • comparer différents vecteurs ou des lots d'un même vecteur obtenus par un même procédé de préparation ou par des procédés de préparation différents.

En variante, ce sont les cellules cibles qui sont soumises à des dilutions en série, R1 étant constante ; les étapes (a) et (b) sont donc modifiées en conséquence et l'étape (d). comprend l'établissement d'une courbe théorique H la mieux ajustée aux valeurs expérimentales (P et R2) par itération, en attribuant des valeurs aux différents paramètres de l'équation de Hill ($P_{max}$, $\kappa$, $\pi$ et r).

La présente invention a également pour objet, un agent biologique modifié caractérisé en ce qu'il est susceptible d'être obtenu par le procédé de criblage selon l'invention.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

- la figure 1A représente les courbes théoriques de Hill (H) obtenues par itération à partir des valeurs expérimentales P et R1 (f(logdil)) des deux échantillons 1 et 2 de rAAV. Les paramètres $\kappa$, $\pi$, $\tau$ et $P_{max}$ de ces deux échantillons ont été déterminés directement à partir de ces courbes théoriques ;
- la figure 1B représente la courbe r = f(logdil) qui permet de déterminer l'index d'hétérogénéité du système biologique ($\eta$) ;
- la figure 2 représente le plot de Hill (log| P/(1-P)| = f(R1)) ;
- la figure 3 illustre l'obtention du paramètre $\varepsilon$ ;
- la figure 4 représente les valeurs expérimentales obtenues (P et R1) et l'ensemble des valeurs théoriques calculées qui ont servi au tracé des différentes courbes présentées dans les figures 1 à 3 ;
- la figure 5 représente les valeurs des différents paramètres de Hill obtenues selon l'invention pour les deux échantillons de rAAV étudiés ;
- la figure 6 (A et B) illustre un système biologique hétérogène ($\eta$ = 2). La figure 6A représente les courbes théoriques de Hill s'ajustant aux valeurs expérimentales de P et de R1 (f(concentration)) ; la figure 6B comprend en abscisse le n° du point, ordonné selon les concentrations de la figure 6A et en ordonnées les valeurs de r ; cette courbe présente 2 paliers ($\eta$ = 2) ;
- la figure 7 illustre le criblage d'une banque de vecteurs d'expression du gène *rep* de rAAV, chacun des vecteurs codant pour un mutant différent dudit gène. Les courbes expérimentales P = f(log 1/dilution) sont présentées pour chaque vecteur et la valeur du titre apparent $\tau$, déterminée à partir de chaque courbe théorique de Hill, est indiquée par une flèche sur l'axe des abscisses;

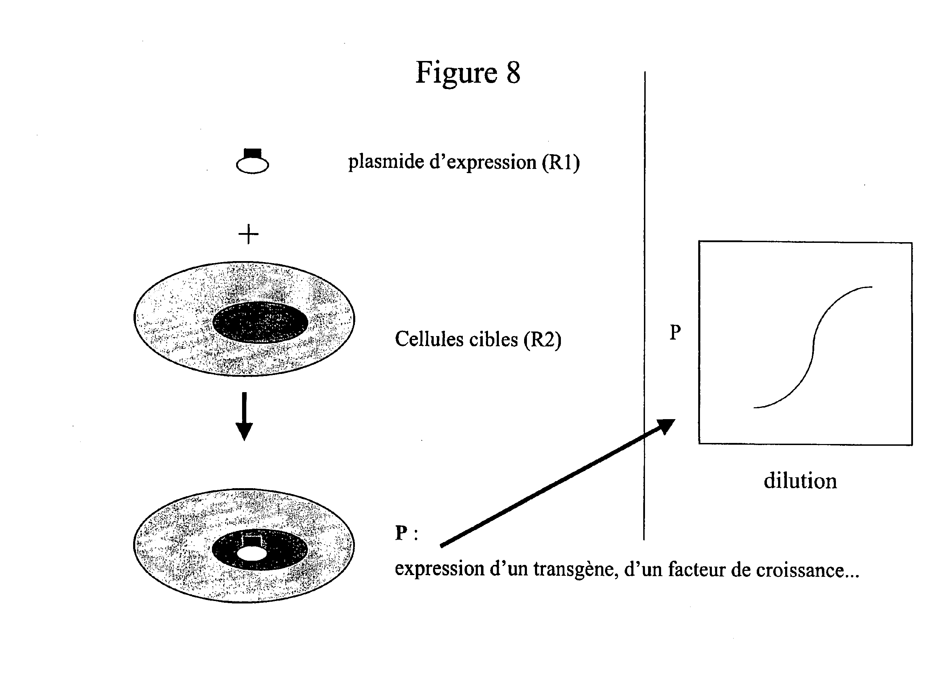

- la figure 8 illustre l'évaluation de la performance d'un plasmide d'expression par le procédé selon l'invention ; et

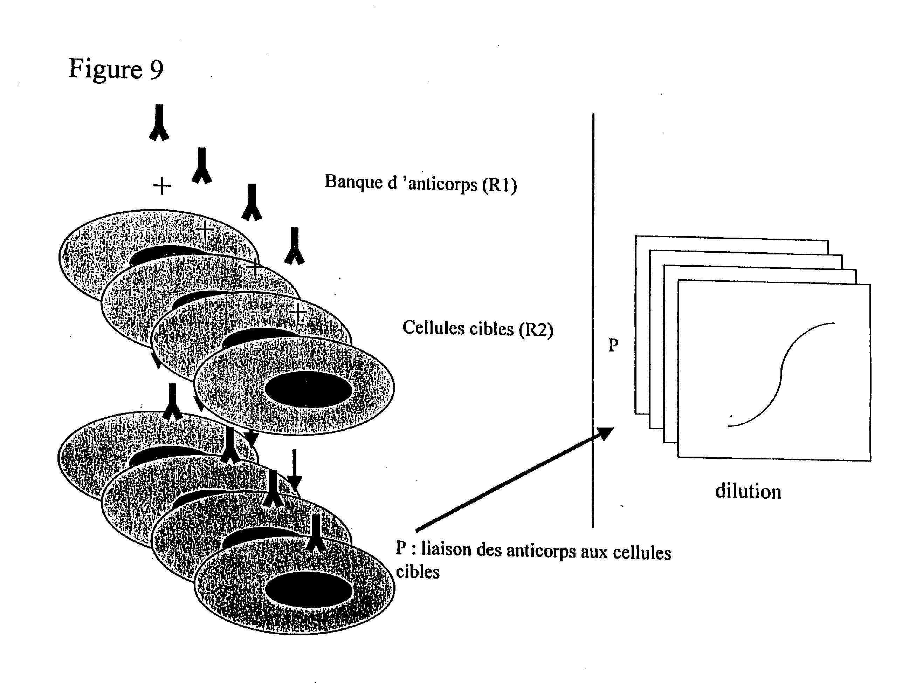

- la figure 9 illustre le criblage d'une banque d'anticorps par le procédé selon l'invention.

Il doit être bien entendu, toutefois, que ces exemples sont donnés uniquement à titre d'illustration de l'objet de l'invention, dont ils ne constituent en aucune manière une limitation.

**Exemple 1 : Comparaison de la performance de deux échantillons de vecteurs recombinants [virus associé à l'adénovirus recombinant (rAAV)] dans des cellules HeLa rc-32.**

a) Matériel et méthode

**- Définition du système biologique**

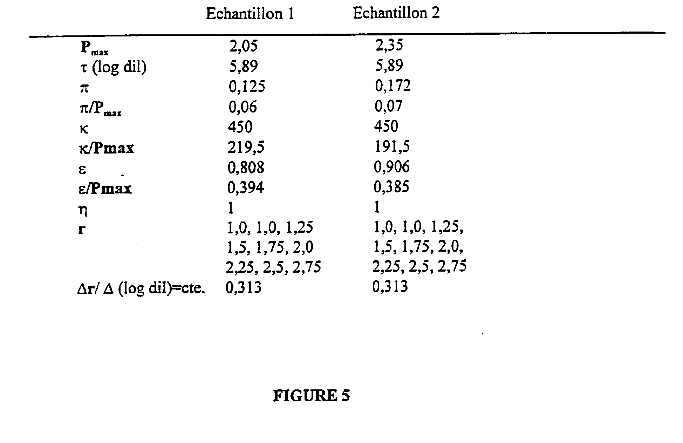

L'agent biologique étudié est un vecteur viral recombinant (rAAV) Deux stocks de rAAV (échantillon 1 et échantillon 2), obtenus selon les techniques classiques connues de l'homme du métier, décrites dans E.M. Atkinson et al. (NAR, 1998, 26, 11, 2821-2823) ont été évalués dans les cellules HeLa rc-32 (A. Salvetti et al., Hum. Gene Ther., 1998, 20, 9, 5, 695-706). Les cellules ont été ensemencées dans les puits d'une plaque de microtitration à une concentration R2 constante, puis infectées avec les dilutions en série des échantillons 1 et 2. Au temps t=48h à 72h, les cellules ont été récoltées puis le génome viral a été isolé et hybridé avec une sonde nucléotidique spécifique marquée, selon la technique du Dot Blot classiquement utilisée par l'homme du métier, décrite dans E.M. Atkinson et al. (NAR, 1998, 26, 11, 2821-2823). Le signal (produit P) représentant la quantité d'ADN hybridée a été mesuré pour chaque dilution des échantillons 1 et 2, à l'aide d'un phosphorimageur et les valeurs obtenues sont présentées dans la figure 4. Le test biologique mis en oeuvre (hybridation) permet d'évaluer la réplication de l'agent biologique (rAAV) dans les cellules cibles HeLa rc-32.

Les étapes suivies, conformément au procédé selon l'invention pour analyser lesdits rAAV sont présentées ci-dessous. L'ensemble des valeurs des différents paramètres de Hill obtenues pour les échantillons 1 et 2 de rAAV est résumé dans la figure 5.

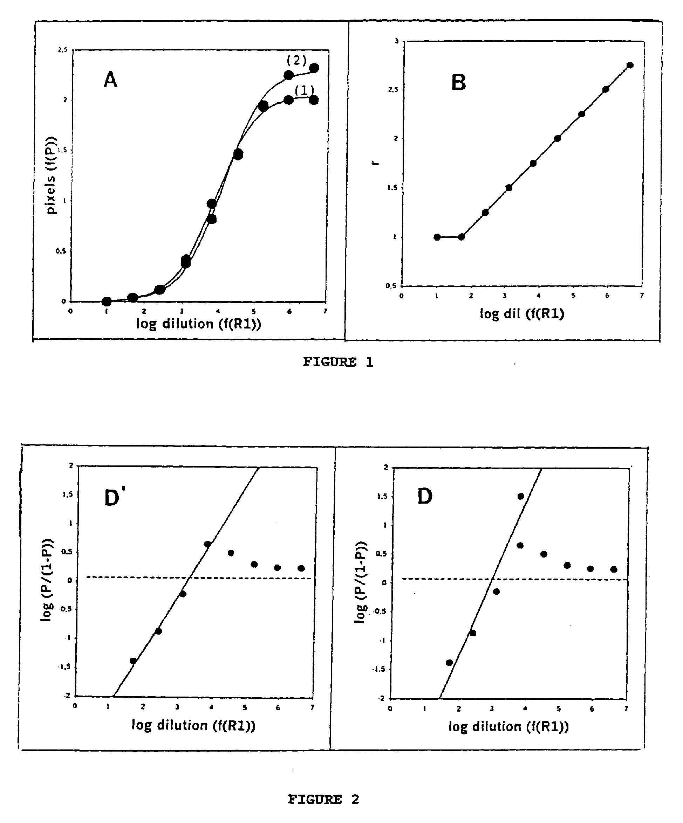

**- Etape 1 : Détermination de la courbe de Hill optimale (H) pour chaque préparation de vecteur (figure 1A)**

La courbe de Hill optimale s'ajustant le mieux avec les valeurs expérimentales a été obtenue par itération en attribuant des valeurs aux différents paramètres de l'équation de Hill, à savoir : $P_{max}$, $\kappa$, $\pi$, et r. Les meilleures courbes de Hill obtenues pour les échantillons 1 et 2 sont présentées sur la figure 1A, elles correspondent respectivement aux valeurs théoriques H1 et H2 des échantillons 1 et 2, (figure 4). Les valeurs de P sont exprimées en unités arbitraires (pixels) en fonction du log de la dilution du vecteur.

Pour l'échantillon 1, on obtient :

$$P = 2{,}05 \, (0{,}125 \, R1)^r \, / \, (450 + (0{,}125 \, R1)^r)$$

Pour l'échantillon 2, on obtient :

$$P = 2{,}35 \, (0{,}172 \, R1)^r \, / \, (450 + (0{,}172 \, R1)^r)$$

\* Avec r=1,0, 1,0, 1,25, 1,5, 1,75, 2,0, 2,25, 2,5, 2,75
\* Le système utilisé pour ce test (les cellules HeLa rc-32) est supposé montrer la même résistance vis à vis des deux échantillons( $\kappa$=450).

**- Etape 2 : Tracé du plot de Hill (figure 2A et 2B)**

Le plot de Hill correspondant à log |P/(1-P)| en fonction du log de la dilution a été tracé, à partir des valeurs expérimentales, pour les deux échantillons[figures 2D (échantillon 1) et 2D' (échantillon 2)]. La régression linéaire obtenue montre que les données présentées dans la figure 1A répondent bien à l'équation de Hill.

**- Etape 3 : Détermination de la concentration limite (titre $\tau$) des préparations de vecteur (figure 1A)**

$\tau$ est déterminé par la valeur maximale de R1 pour laquelle le coefficient de Hill (sur la courbe optimale de Hill) est égal à 1. Les valeurs $\tau$ des échantillons 1 et 2 ont été déterminées à partir des courbes de la figure 1A, les valeurs obtenues sont de 5,89 pour les deux échantillons.

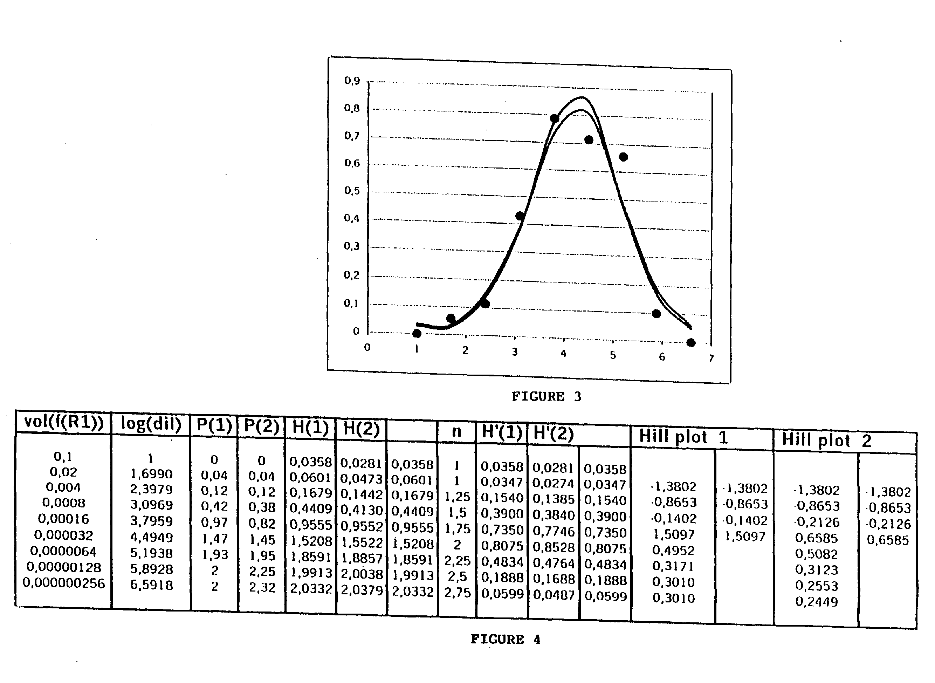

**- Etape 4 : Détermination de l'efficacité ($\varepsilon$) et de l'efficacité standardisée ($\varepsilon$/P$_{max}$) (figure 3)**

L'efficacité est la pente de la courbe de Hill (ou de toute courbe sigmoïdale traduisant les valeurs obtenues) à son point d'inflexion.
$\varepsilon$ a été calculée comme suit :
La courbe H' (H'=$\delta$H/$\delta$R1), dérivée de H a été tracée.
La valeur maximale atteinte par la courbe correspondant à $\varepsilon$ a été déterminée pour les échantillons 1 et 2, les valeurs obtenues sont 0, 808 et 0,906, respectivement (figure 3).

**- Etape 5 : Détermination de l'homogénéité du système biologique ($\eta$), (figure 1B)**

La courbe r en fonction de log dil a été tracée et le nombre de paliers sur la courbe a été déterminé.
L'index d'hétérogénéité $\eta$ a été déterminé comme suit, en fonction du nombre de paliers (un palier ; $\eta$=1, deux paliers ; $\eta$=2, x paliers ; $\eta$=x)
On obtient $\eta$=1; par conséquent le système biologique testé est homogène.

**- Etape 6 : Caractérisation complète du vecteur (figure 5)**

L'ensemble des valeurs obtenues pour chacun des paramètres suivants :

**P$_{max}$**, $\kappa$ **r**, $\varepsilon$, $\tau$, $\pi$, $\eta$, $\varepsilon$/P$_{max}$, $\pi$/P$_{max}$ **et** $\kappa$/P$_{max}$, permet de caractériser chaque préparation de vecteur.

**- Etape 7 : Classement des vecteurs en fonction de leur performance (figure 5)**

Les vecteurs ont été classés en fonction des valeurs obtenues pour :

$$P_{max}, \kappa, \pi, \tau, \pi/P_{max} \text{ et } \kappa/P_{max.}$$

b) Résultats

Les deux échantillons montrent des titres apparents équivalents ($\tau$=5,89); cependant les titres absolus sont différents $\theta_1/\theta_2 = \pi_2/\pi_1 = 0,725$ et la puissance de l'échantillon 2 ($\pi_1$=0,172) est supérieure à celle de l'échantillon 1 ($\pi_2$=0,125).
Cette valeur n'est pas compensée par la puissance corrigée $\pi$/P$_{max}$ puisque l'on retrouve une valeur pour l'échantillon 2 ($\pi$/P$_{max}$=0,07) supérieure à celle de l'échantillon 1 ($\pi$/P$_{max}$=0,06).
En considérant que $\kappa$ est constant, c'est-à-dire que les cellules HeLa rc-32 montrent la même résistance vis-à-vis des deux échantillons, l'échantillon 2 est légèrement plus efficace que l'échantillon 1 ($\varepsilon_2$=0,906>$\varepsilon_1$= 0,808) et sa capacité maximale P$_{max}$=2,35 est supérieure à celle de l'échantillon 1 (P$_{max}$=2,05).
L'analyse des valeurs des différents paramètres de Hill obtenues pour les deux échantillons de vecteur recombinant permet de tirer les conclusions suivantes :

Les titres apparents de deux lots d'un même vecteur sont équivalents et correspondent à ce qui est généralement déterminé dans l'Art antérieur, alors que la détermination des paramètres de Hill et éventuellement des paramètres dérivés permet de mettre en évidence des différences dans leur puissance corrigée et leur efficacité. La caractérisation de deux lots d'un même vecteur viral, à l'aide des paramètres pertinents de Hill et éventuellement de ses paramètres dérivés permet donc bien de valider et d'optimiser des agents biologiques (préparations de vecteurs viraux par exemple) utilisables dans une application particulière (thérapie génique *in vivo*).

**Exemple 2 : Système biologique présentant une hétérogénéité.**

a) Définition du système biologique

L'agent biologique analysé est un vecteur rétroviral dénommé (pSI-EGFP ; Ropp et al., Cytometry, 1995, **21**, 309-317), codant le gène rapporteur de la protéine fluorescente eucaryote *(Eukaryotic Green Fluorescent Protein* ou EGFP) et les cellules cibles sont les cellules HT-1080 (ATCC n° CCL-121).
Les cellules cibles ont été ensemencées à une concentration R2 et infectées avec les dilutions en série du vecteur. Au temps t=48h, l'intensité de fluorescence (produit P) représentant la quantité du gène rapporteur EGFP exprimée par le vecteur a été mesurée pour chaque dilution.

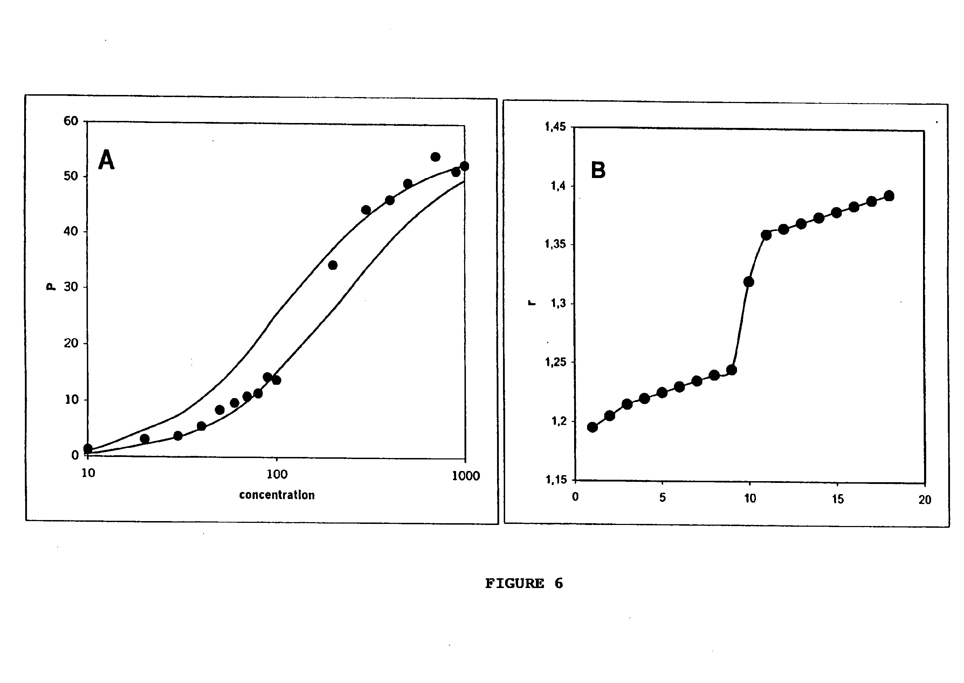

b) Détermination de la courbe de Hill optimale (figure 6A)

L'ensemble des points expérimentaux ne peut pas s'ajuster à une seule courbe de Hill. On observe deux sous-ensembles de points :

- les points situés dans la partie gauche de la figure 6A s'ajustent à une courbe de Hill correspondant à $\kappa$=8000 (courbe supérieure),
- les points situés dans la partie droite de la figure 6A s'ajustent à une courbe de Hill correspondant à $\kappa$=3500 (courbe inférieure).

c) Détermination de l'homogénéité du système biologique ($\eta$), (figure 6B)

La courbe r en fonction des n° de points, ordonnés selon les concentrations telles qu'elles apparaissent à la figure 6A, a été tracée et le nombre de paliers sur la pente de la courbe a été déterminé (figure 6B).
La courbe présente 2 paliers ; par conséquent le système biologique testé est hétérogène et présente un index d'hétérogénéité $\eta$=2.

**Exemple 3 :** Criblage d'une banque de plasmides d'expression de mutants du gène *rep* de rAAV.

a) Matériel et Méthode

**- Définition du système biologique**

L'ensemble d'agents biologiques modifiés est une banque de vecteurs d'expression de mutants du gène *rep* de rAAV et les cellules cibles sont une lignée d'encapsidation de rAAV (lignée HeLa rc-32). Les cellules ont été ensemencées à une concentration constante R2 puis transfectées avec les dilutions en série des différents plasmides d'expression. Au temps t = 48 h, la quantité de virus produite (produit P) a été mesurée selon la technique de dot-blot, telle que décrite à l'exemple 1.

**- Détermination des paramètres de Hill**

Ces paramètres sont déterminés en suivant les étapes 1 à 7 telles que décrites à l'exemple 1 et l'étape finale de sélection du mutant le plus adapté à la production du virus recombinant est effectuée en fonction des valeurs de $\tau$ obtenues.

b) Résultats

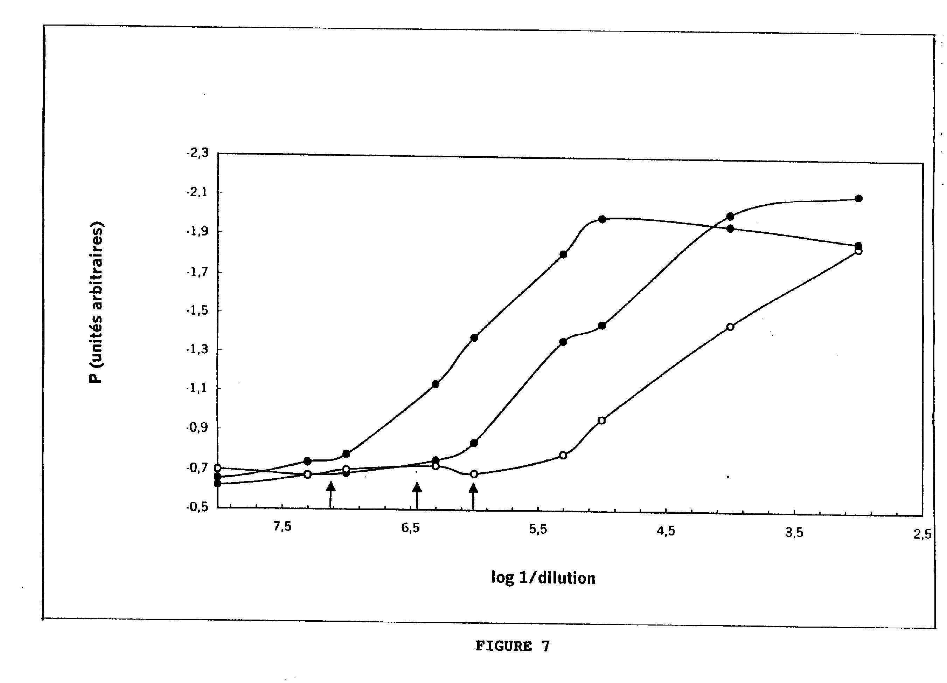

Les courbes expérimentales P = f (log 1/dilution) sont présentées à la figure 7 et le titre apparent $\tau$ de chaque plasmide d'expression, déterminé à partir de la courbe théorique de Hill est le suivant :

- Plasmide n°1 (courbe (1)) : $\tau$ = 7,1
- Plasmide n°2 (courbe (2)) : $\tau$ = 6,4
- Plasmide n°3 (courbe (3)) : $\tau$ = 6

Les résultats indiquent que le mutant du gène *rep* de rAAV contenu dans le plasmide n°1 possède l'activité la plus élevée dans le système testé.

**Revendications**

1. Procédé d'évaluation de la performance d'un ensemble d'agents biologiques complexes, dans des cellules cibles vivantes avec lesquelles lesdits agents biologiques interagissent, lequel procédé est **caractérisé en ce qu'**il comprend au moins les étapes suivantes à partir d'un ensemble d'agents biologiques sélectionné dans le groupe constitué par les virus, les vecteurs viraux et non-viraux, les vaccins, les anticorps et les protéines recombinantes :

(a) la préparation, pour chaque agent biologique dudit ensemble, d'une gamme d'échantillons, obtenue par dilutions en série dudit agent biologique à une concentration R1,

(b) l'incubation de chaque échantillon de ladite gamme de dilutions obtenue en (a) avec lesdites cellules cibles à une concentration constante R2,

(c) la détermination du produit P de la réaction dudit agent biologique à une concentration R1 avec lesdites cellules cibles à la concentration R2, à un instant t, dans chacun desdits échantillons,

(d) l'établissement d'une courbe théorique H à partir desdits points expérimentaux R1 et P, pour chaque agent biologique par approximation itérative de paramètres qui reflètent la réaction R1+R2 → P, audit instant t, conformément à l'équation suivante :

$$P = P_{max} \, (\pi R1)^r \, / \, (\kappa + (\pi R1)^r) \qquad r = 1, ..., n \qquad (2),$$

dans laquelle :

R1 représente la concentration en agent biologique dans un échantillon de la gamme,

P représente le produit de la réaction R1 + R2 à un instant t,

$P_{max}$ représente la capacité maximale de la réaction,

$\kappa$ représente la résistance des cellules cibles vivantes à répondre audit agent biologique,

r représente un coefficient qui dépend de R1 et qui correspond au coefficient de Hill, et

$\pi$ représente la puissance intrinsèque de l'agent biologique à induire une réponse dans les cellules cibles vivantes, et

(e) le tri des valeurs de $\kappa$ et de $\pi$ obtenues en (d), pour chaque agent biologique et le classement des agents biologiques en fonction desdites valeurs.

2. Procédé de criblage d'un ensemble d'agents biologiques complexes modifiés (banque de mutants) dans des cellules cibles vivantes avec lesquelles lesdits agents biologiques interagissent, lequel procédé est **caractérisé en ce qu'**il comprend au moins les étapes (a) à (e) telles que définies à la revendication 1 et une étape (f) de sélection de l'agent biologique le plus adapté à l'application recherchée.

3. Procédé selon la revendication 1 ou la revendication 2, **caractérisé en ce qu'**à l'étape (c), la détermination de P est réalisée soit de manière directe, par exemple par dosage de P, soit de manière indirecte, par exemple, à l'aide d'un test biologique convenablement sélectionné pour la mesure d'au moins un paramètre ou une variable reflétant la réponse du système biologique audit agent biologique.

4. Procédé selon l'une quelconque des revendications 1 à 3, **caractérisé en ce qu'**il comprend, en outre, la mesure d'au moins l'un des paramètres dérivés suivants :

- l'efficacité globale $\varepsilon$ de la réaction induite par l'agent biologique sur ledit système,
- le titre apparent $\tau$ de l'agent biologique, correspondant à l'origine de la courbe théorique H obtenue en (d),
- le titre absolu $\theta$ dudit agent biologique défini par l'équation $\theta\pi = \tau/s$ (3), dans laquelle s représente la sensibilité de la méthode de détection, et
- l'index d'hétérogénéité $\eta$ de ladite réaction biologique.

5. Procédé selon l'une quelconque des revendications 1 à 4, **caractérisé en ce que** la mesure du paramètre d'évaluation $\varepsilon$ représentant l'efficacité spécifique d'un agent biologique apte à induire la production de P dans les cellules cibles vivantes est réalisée soit par mesure de la pente de la courbe théorique obtenue en (d) à son point d'inflexion, soit par calcul du maximum de la dérivée première et éventuellement de la dérivée seconde de la courbe théorique obtenue en (d).

6. Procédé selon l'une quelconque des revendications 1 à 5, **caractérisé en ce qu'**il comprend en outre la mesure des paramètres suivants : $\pi/P_{max}$, $\kappa/P_{max}$ ou $\varepsilon/P_{max}$.

7. Procédé selon l'une quelconque des revendications 1 à 6, **caractérisé en ce que** les valeurs des paramètres de Hill correspondant à chaque agent biologique sont comparées à celles obtenues avec un agent biologique de référence.

**8.** Procédé selon l'une quelconque des revendications 1 à 7, **caractérisé en ce que** pour valider l'analyse de la réaction R1+R2 par l'équation de Hill, le procédé peut comprendre en outre une étape de traitement des données expérimentales obtenues à l'étape (d) (Hill plot), conformément à l'équation suivante :

$$\log|P/(1 - P)| \text{ vs. } \log R1.$$

**9.** Procédé selon l'une quelconque des revendications 1 à 8, **caractérisé en ce qu'**il comprend :

. la sélection des agents biologiques présentant des valeurs minimales acceptables pour les paramètres sélectionnés : $\pi$, $\kappa$, $r$, $\varepsilon$, $\theta$, $\tau$, $\eta$, $\pi/P_{max}$, $\kappa/P_{max}$ ou $\varepsilon/P_{max}$ et
. l'analyse itérative des courbes H correspondant auxdits agents biologiques sélectionnés, conformément à l'équation (2), pour éliminer les valeurs élevées par compensation, suivie de
. l'élimination des agents biologiques qui compensent et pour lesquels des courbes alternatives peuvent être obtenues, pour la constitution d'une liste d'agents biologiques à action optimale.

**10.** Procédé selon l'une quelconque des revendications 2 à 9, **caractérisé en ce que** ledit ensemble d'agents biologiques modifiés est constitué par une banque de mutants, obtenue par l'introduction naturelle ou artificielle d'une ou plusieurs mutations dans la séquence nucléotidique et/ou peptidique desdits agents biologiques.

**Claims**

**1.** A method for assessing the performance of a collection of complex biological agents in living target cells with which said biological agents interact, which method is **characterized in that** it comprises at least the following steps from a collection of biological agents selected from the group consisting of viruses, viral and non viral vectors, vaccines, antibodies and recombinant proteins:

(a) preparing, for each biological agent in said collection, a range of samples, which range is obtained by making serial dilutions of said biological agent at a concentration R1,
(b) incubating each sample of said range of dilutions obtained in (a) with said target cells at a constant concentration R2,
(c) determining the product P of the reaction of said biological agent at a concentration R1 with said target cells at a concentration R2, at a time t, in each of said samples,
(d) constructing a theoretical H curve from said experimental points R1 and P, for each biological agent by iterative approximation of parameters which reflect the reaction R1+R2 $\rightarrow$ P, at said time t, in accordance with the following equation:

$$P = P_{max}(\pi R1)^r/(\kappa + (\pi R1)^r) \qquad r=1,\ldots,n \qquad (2),$$

in which:

R1 represents the concentration of biological agent in a sample of the range,
P represents the product of the reaction R1 + R2 at a time t,
$P_{max}$ represents the maximum capacity of the reaction,
$\kappa$ represents the resistance of the living target cells to responding to said biological agent,
r represents a coefficient which depends on R1 and which corresponds to the Hill coefficient, and
$\pi$ represents the intrinsic power of the biological agent to induce a response in the living target cells, and

(e) sorting the values of $\kappa$ and $\pi$ obtained in (d) for each biological agent and classifying the biological agents in accordance with said values.

**2.** A method for screening a collection of modified complex biological agents (library of mutants) in living target cells with which said biological agents interact, which method is **characterized in that** it comprises at least the steps (a) to (e) as defined in claim 1 and a step (f) of selecting the biological agent which is most suited for the sought-after application.

3. The method as claimed in claim 1 or claim 2, **characterized in that** P is determined in step (c) either directly, for example by assaying P, or indirectly, for example using a biological test which is appropriately selected for measuring at least one parameter or one variable which reflects the response of the biological system to said biological agent.

4. The method as claimed in any one of claims 1 to 3, **characterized in that** it additionally comprises measuring at least one of the following derived parameters:

    - the overall efficiency $\varepsilon$ of the reaction induced by the biological agent on said system,
    - the apparent titer $\tau$ of the biological agent, corresponding to the origin of the theoretical H curve obtained in (d),
    - the absolute titer $\theta$ of said biological agent defined by the equation $\theta\pi = \tau/s$ (3), in which s represents the sensitivity of the detection method, and
    - the heterogeneity index $\eta$ of said biological reaction.

5. The method as claimed in any one of claims 1 to 4, **characterized in that** the measurement of the assessment parameter $\varepsilon$, representing the specific efficiency of a biological agent which is capable of inducing the production of P in the living target cells, is effected either by measuring the slope of the theoretical curve obtained in (d) at its point of inflexion, or by calculating the maximum of the first derivative and, where appropriate, of the second derivative of the theoretical curve obtained in (d).

6. The method as claimed in any one of claims 1 to 5, **characterized in that** it additionally comprises measuring the following parameters: $\pi/P_{max}$, $\kappa/P_{max}$ or $\varepsilon/P_{max}$.

7. The method as claimed in any one of claims 1 to 6, **characterized in that** the values of the Hill parameters corresponding to each biological agent are compared with those obtained using a reference biological agent.

8. The method as claimed in any one of claims 1 to 7, **characterized in that**, in order to validate the analysis of the reaction R1+R2 by the Hill equation, the method can additionally comprise a step of treating the experimental data obtained in step (d) (Hill plot) in accordance with the following equation:

$$\log|P/(1-P)| \text{ vs. } \log R1.$$

9. The method as claimed in any one of claims 1 to 8, **characterized in that** it comprises:

    - selecting the biological agents which exhibit the minimum acceptable values for the selected parameters: $\pi$, $\kappa$, r, $\varepsilon$, $\theta$, $\tau$, $\eta$, $\pi/P_{max}$, $\kappa/P_{max}$ or $\varepsilon/P_{max}$ and
    - iteratively analyzing the H curves corresponding to said selected biological agents, in accordance with equation (2), in order to eliminate the values raised by compensation, followed by
    - eliminating the biological agents which compensate and for which alternative curves can be obtained, in order to form a list of biological agents having an optimal action.

10. The method as claimed in any one of claims 2 to 9, **characterized in that** said collection of modified biological agents consists of a library of mutants which is obtained by naturally or artificially introducing one or more mutations into the nucleotide and/or peptide sequence of said biological agents.

**Patentansprüche**

1. Verfahren zur Bewertung der Leistung einer Einheit von komplexen biologischen Wirkstoffen in lebenden Zielzellen, mit denen die biologischen Wirkstoffe interagieren, wobei das Verfahren **dadurch gekennzeichnet ist, dass** es mindestens die folgenden Schritte ausgehend von einer Einheit von biologischen Wirkstoffen aufweist, die aus der Gruppe ausgewählt werden, die aus den Viren, den viralen Vektoren und den nicht-viralen Vektoren, den Impfstoffen, den Antikörpern und den rekombinierenden Proteinen besteht:

    (a) für jeden biologischen Wirkstoff der Einheit die Herstellung einer Reihe von Proben, die durch Serienverdünnungen des biologischen Wirkstoffs mit einer Konzentration R1 erhalten wird,

(b) die Inkubation jeder Probe der in (a) erhaltenen Reihe von Verdünnungen mit den Zielzellen mit einer konstanten Konzentration R2,

(c) die Bestimmung des Produkts P der Reaktion des biologischen Wirkstoffs mit einer Konzentration R1 mit den Zielzellen mit der Konzentration R2 in einem Zeitpunkt t in jeder der Proben,

(d) die Erstellung einer theoretischen Kurve H ausgehend von den experimentalen Punkten R1 und P für jeden biologischen Wirkstoff durch iterative Approximation von Parametern, die die Reaktion R1+R2 → P im Zeitpunkt t reflektieren, gemäß der folgenden Gleichung:

$$P = P_{max}(\pi R1)^r/(\kappa + (\pi R1)^r) \qquad r=I, \ldots, n \qquad (2),$$

in der:

R1 die Konzentration an biologischem Wirkstoff in einer Probe der Reihe darstellt,

P das Produkt der Reaktion R1 + R2 in einem Zeitpunkt t darstellt,

$P_{max}$ die maximale Kapazität der Reaktion darstellt,

$\kappa$ den Widerstand der lebenden Zielzellen darstellt, auf den biologischen Wirkstoff zu reagieren,

r einen Koeffizienten darstellt, der von R1 abhängt und der dem Hill-Koeffizienten entspricht, und

$\pi$ die wahre Leistung des biologischen Wirkstoffs darstellt, eine Reaktion in die lebenden Zielzellen zu induzieren, und

(e) das Sortieren der in (d) erhaltenen Werte von $\kappa$ und von $\pi$ für jeden biologischen Wirkstoff und das Einordnen der biologischen Wirkstoffe in Abhängigkeit von den Werten.

2. Verfahren zur Klassierung einer Einheit von komplexen modifizierten biologischen Wirkstoffen (Mutantenbank) in lebenden Zielzellen, mit denen die biologischen Wirkstoffe interagieren, wobei das Verfahren **dadurch gekennzeichnet ist, dass** es mindestens die Schritte (a) bis (e) wie in Anspruch 1 definiert und einen Schritt (f) der Auswahl des am besten für die gesuchte Anwendung geeigneten biologischen Wirkstoffs aufweist.

3. Verfahren nach Anspruch 1 oder Anspruch 2, **dadurch gekennzeichnet, dass** im Schritt (c) die Bestimmung von P entweder direkt, zum Beispiel durch Dosierung von P, oder indirekt, zum Beispiel mit Hilfe eines geeignet ausgewählten biologischen Tests für die Messung mindestens eines Parameters oder einer Variablen durchgeführt wird, der die Reaktion des biologischen Systems auf den biologischen Wirkstoff reflektiert.

4. Verfahren nach einem der Ansprüche 1 bis 3, **dadurch gekennzeichnet, dass** es außerdem die Messung mindestens eines der folgenden abgeleiteten Parameter aufweist:

- die globale Effizienz $\varepsilon$ der durch den biologischen Wirkstoff in das System induzierten Reaktion,
- der apparente Titer $\tau$ des biologischen Wirkstoffs, der dem Ursprung der in (d) erhaltenen theoretischen Kurve H entspricht,
- der absolute Titer $\theta$ des biologischen Wirkstoffs, der durch die Gleichung $\theta\pi=\tau/s$ (3) erhalten wird, in der s die Empfindlichkeit der Erfassungsmethode darstellt,
- der Heterogenitätsindex $\eta$ der biologischen Reaktion.

5. Verfahren nach einem der Ansprüche 1 bis 4, **dadurch gekennzeichnet, dass** die Messung des Bewertungsparameters $\varepsilon$, der die spezifische Effizienz eines biologischen Wirkstoffs darstellt, der in der Lage ist, die Produktion von P in die lebenden Zielzellen zu induzieren, entweder durch Messung der Neigung der in (d) erhaltenen theoretischen Kurve an ihrem Wendepunkt oder durch Berechnung des Maximums der ersten Ableitung und ggf. der zweiten Ableitung der in (d) erhaltenen theoretischen Kurve durchgeführt wird.

6. Verfahren nach einem der Ansprüche 1 bis 5, **dadurch gekennzeichnet, dass** es außerdem die Messung der folgenden Parameter aufweist: $\pi/P_{max}$, $\kappa/P_{max}$ oder $\varepsilon/P_{max}$.

7. Verfahren nach einem der Ansprüche 1 bis 6, **dadurch gekennzeichnet, dass** die Werte der Hill-Parameter entsprechend jedem biologischen Wirkstoff mit denjenigen verglichen werden, die mit einem biologischen Referenzwirkstoff erhalten werden.

8. Verfahren nach einem der Ansprüche 1 bis 7, **dadurch gekennzeichnet, dass** zur Validierung der Analyse der Reaktion R1+R2 durch die Hill-Gleichung das Verfahren außerdem einen Schritt der Verarbeitung der experimentellen Daten aufweisen kann, die im Schritt (d) (Hill plot) erhalten werden, gemäß der folgenden Gleichung:

$$\log \left| P/(1-P) \right| \text{ vs. } \log R1.$$

9. Verfahren nach einem der Ansprüche 1 bis 8, **dadurch gekennzeichnet, dass** es aufweist:

. die Wahl der biologischen Wirkstoffe, die akzeptable Mindestwerte für die ausgewählten Parameter: $\pi$, $\kappa$, r, $\varepsilon$, $\theta$, $\tau$, $\eta$, $\pi/P_{max}$, $\kappa/P_{max}$ oder $\varepsilon/P_{max}$ aufweisen, und
. die iterative Analyse der Kurven H entsprechend den ausgewählten biologischen Wirkstoffen gemäß der Gleichung (2), um die hohen Werte durch Kompensation zu eliminieren, gefolgt von
. der Eliminierung der biologischen Wirkstoffe, die kompensieren und für die alternative Kurven erhalten werden können, zur Erstellung einer Liste von biologischen Wirkstoffen mit optimaler Wirkung.

10. Verfahren nach einem der Ansprüche 2 bis 9, **dadurch gekennzeichnet, dass** die Einheit von modifizierten biologischen Wirkstoffen aus einer Mutantenbank besteht, die durch die natürliche oder künstliche Einführung einer oder mehrerer Mutationen in die Nukleotidsequenz und/oder Peptidsequenz der biologischen Wirkstoffe erhalten wird.

FIGURE 1

FIGURE 2

FIGURE 3

| vol((f(R1))) | log(dil) | P(1) | P(2) | H(1) | H(2) | n | H'(1) | H'(2) | Hill plot 1 | | Hill plot 2 | |
|---|---|---|---|---|---|---|---|---|---|---|---|---|
| 0,1 | 1 | 0 | 0 | 0,0358 | 0,0281 | 1 | 0,0358 | 0,0281 | | -1,3802 | | -1,3802 |
| 0,02 | 1,6990 | 0,04 | 0,04 | 0,0601 | 0,0473 | 1 | 0,0347 | 0,0274 | | -0,8653 | | -0,8653 |
| 0,004 | 2,3979 | 0,12 | 0,12 | 0,1679 | 0,1442 | 1,25 | 0,1540 | 0,1385 | -1,3802 | -0,1402 | -1,3802 | -0,2126 |
| 0,0008 | 3,0969 | 0,42 | 0,38 | 0,4409 | 0,4130 | 1,5 | 0,3900 | 0,3840 | -0,8653 | 1,5097 | -0,8653 | 0,6585 |
| 0,00016 | 3,7959 | 0,97 | 0,82 | 0,9555 | 0,9552 | 1,75 | 0,7350 | 0,7746 | -0,1402 | 0,4952 | -0,2126 | 0,5082 |
| 0,000032 | 4,4949 | 1,47 | 1,45 | 1,5208 | 1,5522 | 2 | 0,8075 | 0,8528 | 1,5097 | 0,3171 | 0,6585 | 0,3123 |
| 0,0000064 | 5,1938 | 1,93 | 1,95 | 1,8591 | 1,8857 | 2,25 | 0,4834 | 0,4764 | | 0,3010 | | 0,2553 |
| 0,00000128 | 5,8928 | 2 | 2,25 | 1,9913 | 2,0038 | 2,5 | 0,1888 | 0,1688 | | 0,3010 | | 0,2449 |
| 0,000000256 | 6,5918 | 2 | 2,32 | 2,0332 | 2,0379 | 2,75 | 0,0599 | 0,0487 | | | | |

FIGURE 4

| | Echantillon 1 | Echantillon 2 |
|---|---|---|
| $P_{max}$ | 2,05 | 2,35 |
| $\tau$ (log dil) | 5,89 | 5,89 |
| $\pi$ | 0,125 | 0,172 |
| $\pi/P_{max}$ | 0,06 | 0,07 |
| $\kappa$ | 450 | 450 |
| $\kappa/Pmax$ | 219,5 | 191,5 |
| $\varepsilon$ | 0,808 | 0,906 |
| $\varepsilon/Pmax$ | 0,394 | 0,385 |
| $\eta$ | 1 | 1 |
| r | 1,0, 1,0, 1,25 1,5, 1,75, 2,0 2,25, 2,5, 2,75 | 1,0, 1,0, 1,25, 1,5, 1,75, 2,0, 2,25, 2,5, 2,75 |
| $\Delta r/\Delta$ (log dil)=cte. | 0,313 | 0,313 |

**FIGURE 5**

FIGURE 6

FIGURE 7

# Figure 8

plasmide d'expression (R1)

+

Cellules cibles (R2)

**P** :

expression d'un transgène, d'un facteur de croissance...

P

dilution

# Figure 9

Banque d'anticorps (R1)

Cellules cibles (R2)

P

P : liaison des anticorps aux cellules cibles

dilution

EP 1 244 912 B1